# EUROPEAN PATENT APPLICATION

(11) **EP 4 796 040 A1**
(43) Date of publication of application: **26.08.2026**
(21) Application number: 24879009.9
(22) Date of filing: 15.10.2024
(51) Int. Cl.: A61B 6/00, G06T 11/00

(54) **SCATTER CORRECTION METHOD AND SYSTEM**

(30) Priority: 20.10.2023 CN 202311372202
(71) Applicant: Nanovision (Nanjing) Technology Group Co., Ltd., Nanjing, Jiangsu 211102 (CN)
(72) Inventor: QI, Yanjun, Beijing 100094 (CN); HOU, Xiaoran, Beijing 100094 (CN); LI, Yunxiang, Beijing 100094 (CN); DU, Qiang, Beijing 100094 (CN)
(74) Representative: Wang, Bo
(86) International application number: PCT/CN2024/125088
(87) International publication number: WO 2025/082367

(57) **Abstract**

Provided are a scatter correction method and system. The method comprises the following steps: respectively acquiring an irradiation intensity distribution I₀ of an X-ray before passing through an object and an irradiation intensity distribution I of the X-ray after passing through the object (S1); calculating the thicknesses t=I/I₀ of the object at different locations, and dividing the thicknesses of the object into n groups (S2); on the basis of the thickness groups of the object, the irradiation intensity distribution I and an X-ray attenuation index, acquiring irradiation intensity distributions S corresponding to different thickness groups (S3); calculating A on the basis of the thickness groups of the object and the corresponding irradiation intensity distributions S (S4); performing pre-simulation to obtain shape factors F corresponding to different thickness groups of the object (S5); performing a two-dimensional convolution operation between A and the shape factors F, and performing superposition to obtain a scatter distribution ISC of the object (S6); and performing scatter correction on the basis of the scatter distribution ISC of the object (S7). According to the method, the irradiation intensity distributions of the X-ray before and after passing through the object can be used as a basis for grouping the thicknesses of the object, and then the overall scatter distribution of the object is obtained by means of scatter weighting of different thickness groups.

## Description

### BACKGROUND

### Technical Field

The present invention belongs to the field of radiation imaging technologies, and relates to a scatter correction method and a corresponding scatter correction system.

### Related Art

Computed tomography (CT) is a technology that uses an X-ray beam and a detector to scan a cross-section of an object. The technology is to obtain projection data reflecting a physical or chemical property of the cross-section by measuring an absorption coefficient of an X-ray to a human organ or tissue. Then a computer uses the data to calculate a parameter value of any location on the cross-section and finally generate a tomographic image.

In conventional CT scanning, a scatter problem may occur, that is, the X-ray undergoes Compton scattering when passing through the object, causing the detector to receive a scattered ray, thereby reducing a spatial resolution and contrast of the image. This scattering phenomenon may introduce an artifact in the image, such as a "cup-shaped" artifact and a streak artifact, which affects image diagnosis and clinical application.

For a static CT scanning device, due to unique geometric configuration thereof and the design where a plurality of radiation sources irradiate a same detector module, hardware such as a post-collimator or grids is not suitable for scatter reduction. Therefore, a software-based method generally needs to be used to suppress scattering. Through the software-based method, a scatter distribution is estimated by analyzing the projection data, and correction is performed during image reconstruction, to improve image quality and diagnostic accuracy.

### SUMMARY

A primary technical problem to be resolved in the present invention is to provide a scatter correction method.

Another technical problem to be resolved in the present invention is to provide a scatter correction system.

To achieve the foregoing technical objective, the present invention adopts the following technical solutions.

According to a first aspect of embodiments of the present invention, a scatter correction method is provided, including the following steps:
obtaining an irradiation intensity distribution I₀ of an X-ray before passing through an object and an irradiation intensity distribution I of the X-ray after passing through the object;
calculating thicknesses t=I/I₀ of the object at different locations, and dividing the thicknesses of the object into n groups based on a preset thickness grouping boundary value, where n is a positive integer;
obtaining, based on thickness groups of the object, the irradiation intensity distribution I, and an X-ray attenuation index, irradiation intensity distributions S corresponding to different thickness groups, where S ∈(S1, S2, ..., and Sn);
calculating, based on the thickness groups of the object and the irradiation intensity distributions S corresponding to different thickness groups and based on amplitude factors corresponding to different thickness groups, products A of the amplitude factors corresponding to different thickness groups and the irradiation intensity distribution I of the X-ray after passing through the object, where A∈(A1, A2, ..., and An);
obtaining shape factors F corresponding to different thickness groups of the object through simulation in advance, where F∈(F₁, F₂, ..., and Fₙ);
performing a two-dimensional convolution operation on A and the shape factors F corresponding to different thickness groups of the object, to obtain a scatter distribution I_{SC} of the object through superposition; and
performing, based on the scatter distribution I_{SC} of the object, scatter correction on the irradiation intensity distribution I of the X-ray after passing through the object.

Preferably, the performing, based on the scatter distribution I_{SC} of the object, scatter correction on the irradiation intensity distribution I of the X-ray after passing through the object may be replaced by:
performing an iterative operation on the scatter distribution I_{SC} of the object to obtain an iterative scatter distribution I_{sc}(i) of the object, where i refers to a quantity of iterations; and
performing, based on the iterative scatter distribution I_{sc}(i) of the object, scatter correction on the irradiation intensity distribution I of the X-ray after passing through the object.

Preferably, the performing an iterative operation on the scatter distribution I_{SC} of the object to obtain an iterative scatter distribution I_{sc}(i) of the object specifically includes:
obtaining a ray distribution Iₚᵣ(i-1) and the irradiation intensity distribution I₀ before the X-ray passes through the object, where Iₚᵣ(i-1)=I-I_{sc}(i-1), I_{sc}(0)=0, and Iₚᵣ(0)=I;
using Iₚᵣ(0) and I₀ as an input to obtain an iterative scatter distribution I_{sc}(1) of the object through calculation;
obtaining a new ray distribution Iₚᵣ(i) through calculation, where Iₚᵣ(i)=Iₚᵣ(i-1)+λ[I_{sc}(i-1)-I_{sc}(i)], λ is a relaxation factor that controls and adjusts a speed of iterative convergence, and a value of λ ranges from 0.6 to 0.8;
determining whether a current quantity of iterations reaches a preset value;
still performing iteration if the quantity of iterations does not reach the preset value, until the quantity of iterations reaches the preset value, and determining whether convergence is achieved if the quantity of iterations reaches the preset value; and
outputting the currently calculated iterative scatter distribution I_{sc}(i) if the convergence is achieved, and still performing, if the convergence is not achieved, a next iteration to obtain a scatter distribution I_{sc}(i+1), and simultaneously updating Iₚᵣ(i+1) until the convergence is achieved.

Preferably, the determining whether convergence is achieved specifically includes:
obtaining a ratio of a difference between a current iterative scatter distribution I_{sc}(i) and a previous iterative scatter distribution I_{sc}(i-1) to I_{sc}(1);
comparing the ratio with a preset coefficient ε; and
determining that the convergence is achieved if the ratio is less than the preset coefficient ε, and determining that the convergence is not achieved if the ratio is not less than the preset coefficient ε.

Preferably, a convergence degree of the iterative scatter distribution I_{sc}(i) is positively correlated with the quantity of iterations of the iterative scatter distribution I_{sc}(i).

Preferably, the irradiation intensity distributions I₀ and I are downsampled, and the scatter distribution I_{SC} of the object is upsampled after the two-dimensional convolution operation.

Preferably, the iterative operation is performed on the iterative scatter distribution I_{sc}(i) by using a CUDA acceleration algorithm.

Preferably, an exposure mode of the X-ray is alternate exposure of a single X-ray source in static CT.

Preferably, an exposure mode of the X-ray is simultaneous exposure of a plurality of X-ray sources in the static CT.

According to a second aspect of embodiments of the present invention, a scatter correction system is provided, including a processor and a memory, where the processor reads a computer program in the memory to perform the following operations:
obtaining an irradiation intensity distribution I₀ of an X-ray before passing through an object and an irradiation intensity distribution I of the X-ray after passing through the object;
calculating thicknesses t=I/I₀ of the object at different locations, and dividing the thicknesses of the object into n groups based on a preset thickness grouping boundary value, where n is a positive integer;
obtaining, based on thickness groups of the object, the irradiation intensity distribution I, and an X-ray attenuation index, irradiation intensity distributions S corresponding to different thickness groups, where S∈(S1, S2, ..., and Sn);
calculating, based on the thickness groups of the object and the irradiation intensity distributions S corresponding to different thickness groups and based on amplitude factors corresponding to different thickness groups, products A of the amplitude factors corresponding to different thickness groups and the irradiation intensity distribution I of the X-ray after passing through the object, where A∈(A1, A2, ..., and An);
obtaining shape factors F corresponding to different thickness groups of the object through simulation in advance, where F∈(F₁, F₂, ..., and Fₙ);
performing a two-dimensional convolution operation on A and the shape factors F corresponding to different thickness groups of the object, to obtain a scatter distribution I_{SC} of the object through superposition; and
performing, based on the scatter distribution I_{SC} of the object, scatter correction on the irradiation intensity distribution I of the X-ray after passing through the object.

Compared with the prior art, the present invention has the following beneficial effects:
1. The shape factors of scatter kernels for different thickness groups applicable to a full-ring detector are obtained through simulation. This extends a scatter deconvolution method from a conventional wide-fan-beam CT or CBCT scenario to a static CT scenario with a full-ring circular detector structure.
2. The irradiation intensity distributions of the X-ray before and after passing through the object can be used as a basis for grouping the thicknesses of the object, and then an overall scatter distribution of the object is obtained through scatter weighting of different thickness groups.
3. A scanning imaging mode of single-source alternate exposure for static CT and multi-source simultaneous exposure is supported.
4. Downsampling, upsampling, and the CUDA acceleration algorithm are used to accelerate scatter calculation to improve calculation efficiency.

### BRIEF DESCRIPTION OF THE DRAWINGS

FIG. 1 is a flowchart of a scatter correction method according to a first embodiment of the present invention;
FIG. 2 is a schematic diagram of non-iterative scatter correction calculation according to a first embodiment of the present invention;
FIG. 3 is a flowchart of another scatter correction method according to a second embodiment of the present invention;
FIG. 4 is a schematic diagram of iterative scatter correction calculation according to a second embodiment of the present invention; and
FIG. 5 is a structure diagram of a scatter correction system according to a third embodiment of the present invention.

### DETAILED DESCRIPTION

Technical content of the present invention is described in detail below with reference to the accompanying drawings and specific embodiments.

### First embodiment

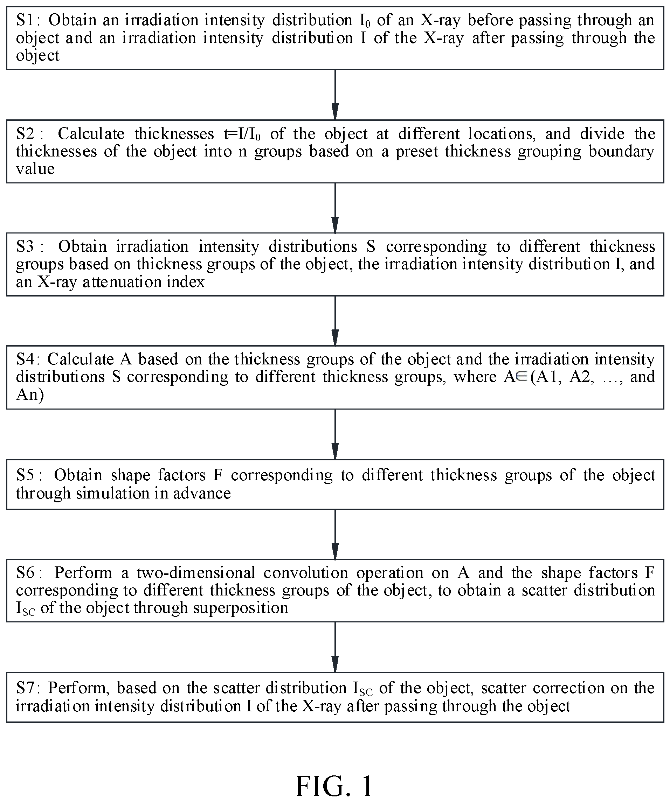

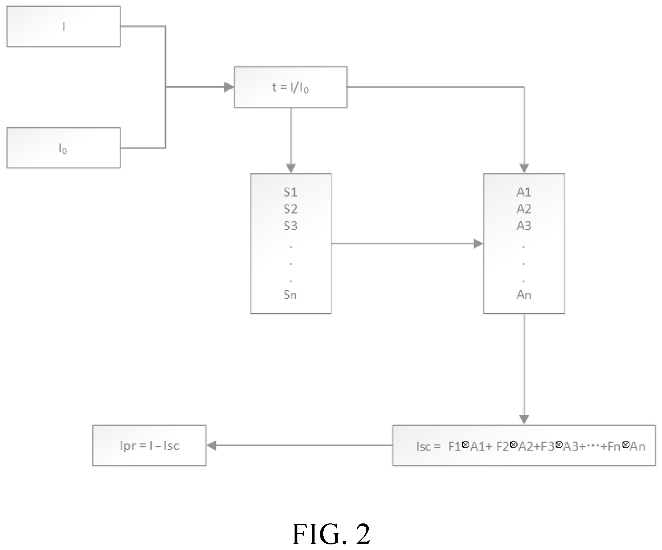

As shown in FIG. 1 and FIG. 2, a scatter correction method provided in the first embodiment of the present invention specifically includes steps S1-S7.

S1: Obtain an irradiation intensity distribution I₀ of an X-ray before passing through an object and an irradiation intensity distribution I of the X-ray after passing through the object.

Specifically, in this embodiment, an exposure mode of the X-ray may be either alternate exposure of a single radiation source in static CT, or simultaneous exposure of a plurality of radiation sources in static CT.

When a CT scan of the object (for example, a human body) is required, the irradiation intensity distribution I₀ of the X-ray before passing through the object is obtained first. Then the object is placed into a CT machine, and then the irradiation intensity distribution I of the X-ray after passing through the object is obtained.

S2: Calculate thicknesses t=I/I₀ of the object at different locations, and divide the thicknesses of the object into n groups based on a preset thickness grouping boundary value.

It may be understood that the irradiation intensity distribution I of the X-ray after passing through the object is necessarily less than the irradiation intensity distribution I₀ of the X-ray before passing through the object. In addition, a thicker object indicates a smaller irradiation intensity distribution I of the X-ray after passing through the object, and conversely, a thinner object indicates a larger irradiation intensity distribution I of the X-ray after passing through the object.

In this way, the thicknesses t=I/I₀ of the object at different locations may be calculated based on the irradiation intensity distributions I₀ and I in step S1, so as to divide the thicknesses of the same object at different locations into n groups based on the preset thickness grouping boundary value, where n is a positive integer.

For example, after the thicknesses t of the object at different locations are calculated based on the irradiation intensity distributions I₀ and I in step S1, thicknesses below 5 cm may be divided into one group, thicknesses ranging from 5 cm to 10 cm may be divided into one group, thickness ranging from 10 cm to 15 cm may be divided into one group, and so on, so as to divide the thicknesses of the same object at different locations into n groups. It may be understood that a specific quantity of groups n may be adaptively adjusted as needed.

S3: Obtain, based on thickness groups of the object, the irradiation intensity distribution I, and an X-ray attenuation index, irradiation intensity distributions S corresponding to different thickness groups.

Specifically, after the irradiation intensity distribution I of the X-ray after passing through the object is obtained based on step S1, and the thickness groups of the object are obtained based on step S2, the irradiation intensity distributions S corresponding to different thickness groups may be calculated based on the X-ray attenuation index.

S ∈ (S₁, S₂, ..., and Sₙ), where S₁ represents the irradiation intensity distribution corresponding to a first thickness group, S₂ represents the irradiation intensity distribution corresponding to a second thickness group, and Sₙ represents the irradiation intensity distribution corresponding to an n^{th} thickness group.

S4: Calculate, based on the thickness groups of the object and the irradiation intensity distributions S corresponding to different thickness groups and based on amplitude factors corresponding to different thickness groups, products A of the amplitude factors corresponding to different thickness groups and the irradiation intensity distribution I of the X-ray after passing through the object, where A∈(A1, A2, ..., and An).

S5: Obtain shape factors F corresponding to different thickness groups of the object through simulation in advance.

In this embodiment, F ∈ (F₁, F₂, ..., and Fₙ), where F₁ represents the shape factor corresponding to the first thickness group, F₂ represents the shape factor corresponding to the second thickness group, and Fₙ represents the shape factor corresponding to the n^{th} thickness group.

S6: Perform a two-dimensional convolution operation on A and the shape factors F corresponding to different thickness groups of the object, to obtain a scatter distribution I_{SC} of the object through superposition.

Specifically, the method includes steps S61 and S62.

S61: Perform the two-dimensional convolution operation on A and the shape factors F corresponding to different thickness groups, to obtain scatter contributions of different thickness groups.

Specifically, in this embodiment, a convolution operation result of the first thickness group is F₁⊗A₁, and the convolution operation result is the scatter contribution of the first thickness group, where ⊗ represents a convolution operation symbol. Similarly, the convolution operation result of the second thickness group is F₂⊗A₂, and the convolution operation result of the n^{th} thickness group is Fₙ⊗Aₙ.

S62: Superpose the scatter contributions of all the thickness groups to obtain the overall scatter distribution I_{SC} of the object. ${I}_{SC} = {F}_{1} ⊗ {A}_{1} + {F}_{2} ⊗ {A}_{2} + … + {F}_{n} ⊗ {A}_{n} .$

S7: Perform, based on the scatter distribution I_{SC} of the object, scatter correction on the irradiation intensity distribution I of the X-ray after passing through the object.

Specifically, after the overall scatter distribution I_{SC} of the object is obtained based on step S6, an X-ray distribution Iₚᵣ without object scattering may be obtained by subtracting the overall scatter distribution I_{SC} of the object from the irradiation intensity distribution I of the X-ray after passing through the object, that is, Iₚᵣ=I-I_{SC}.

In the foregoing embodiment, preferably, in step S1, the irradiation intensity distributions I₀ and I are downsampled first, and in step S6, the scatter distribution I_{SC} after the two-dimensional convolution operation is upsampled. Therefore, calculation efficiency can be significantly improved based on low frequency characteristics of scattering.

### Second embodiment

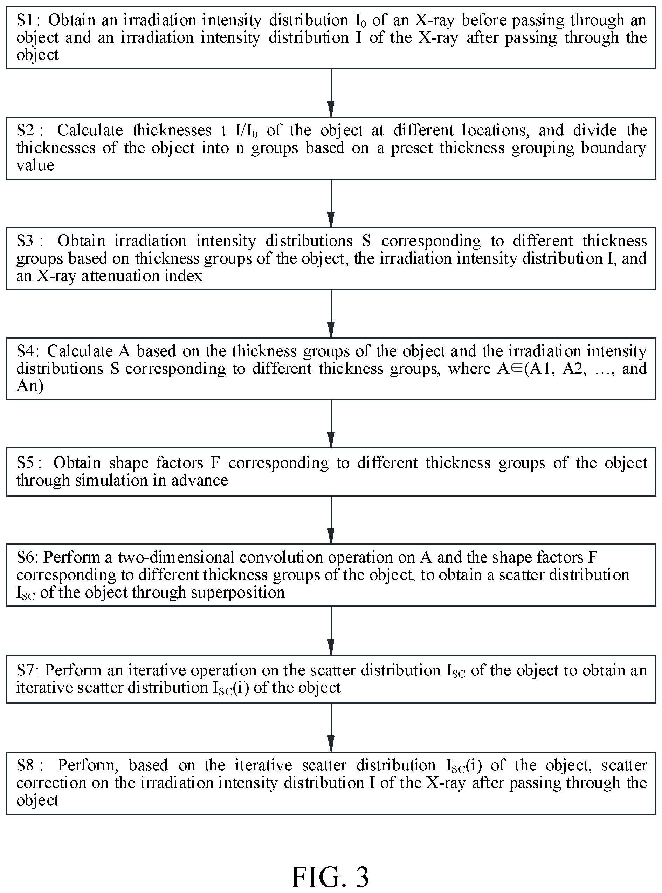

Referring to FIG. 3, based on the foregoing first embodiment, the second embodiment of the present invention further provides another scatter correction method. Compared with the first embodiment, a difference lies in steps after step S6.

Specifically, in this embodiment, after step S6, the method further includes the following steps.

S7: Perform an iterative operation on the scatter distribution I_{SC} of the object to obtain an iterative scatter distribution I_{sc}(i) of the object.

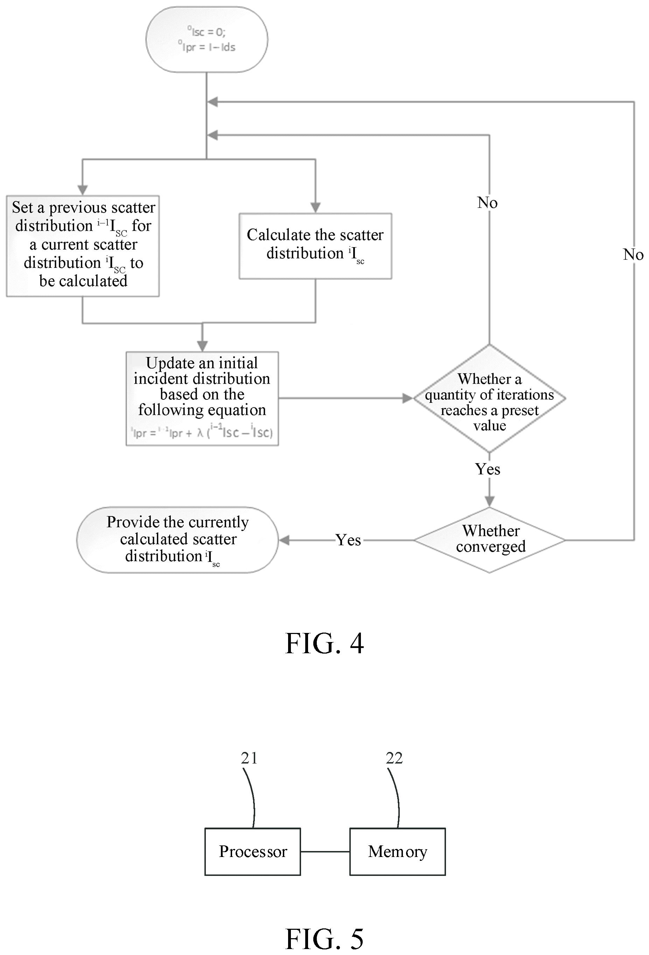

Specifically, as shown in FIG. 4, steps S71-S74 are included.

S71: Obtain a ray distribution Iₚᵣ(i-1) and the irradiation intensity distribution I₀ before the X-ray passes through the object, where Iₚᵣ(i-1)=I-I_{sc}(i-1), I_{sc}(0)=0, and Iₚᵣ(0)=I.

S72: Use Iₚᵣ(0) and I₀ as an input to calculate an iterative scatter distribution I_{sc}(1) of the object.

S73: Obtain a new ray distribution Iₚᵣ(i) through calculation. ${I}_{pr} \left(i\right) = {I}_{pr} \left(i-1\right) + λ \left[{I}_{sc}\left(i-1\right)-{I}_{sc}\left(i\right)\right] ,$ where λ is a relaxation factor that controls and adjusts a speed of iterative convergence, and a value of λ ranges from 0.6 to 0.8.

S74: Determine whether a current quantity of iterations reaches a preset value.

If the quantity of iterations does not reach the preset value, the iteration is still performed until the quantity of iterations reaches the preset value, and if the quantity of iterations reaches the preset value, a next step is performed.

S75: Determine convergence.

The currently calculated iterative scatter distribution I_{sc}(i) is outputted if the convergence is achieved, and if the convergence is not achieved, a next iteration is still performed to obtain a scatter distribution I_{sc}(i+1), and Iₚᵣ(i+1) is simultaneously updated, until the convergence is achieved. It may be understood that, in the foregoing iterative operation process, a convergence degree of the iterative scatter distribution I_{sc}(i) is positively correlated with a quantity of iterations of the iterative scatter distribution I_{sc}(i). In other words, a larger quantity of iterations indicates better convergence of the iterative scatter distribution I_{sc}(i). Without loss of generality, a convergence requirement can be satisfied by setting the quantity of iterations to 5-8 times.

In this embodiment, a ratio of a difference between the current iterative scatter distribution I_{sc}(i) and a previous iterative scatter distribution I_{sc}(i-1) to I_{sc}(1) is compared with a preset coefficient ε, to determine the convergence. If the ratio is less than the preset coefficient ε, it is determined that the convergence is achieved; and if the ratio is not less than the preset coefficient ε, it is determined that the convergence is not achieved. The preset coefficient ε is a real number close to 0 between 0 and 1, and may be set to 0.1, 0.05, 0.01, or the like.

In addition, in the foregoing embodiment, a CUDA acceleration algorithm is used to perform the iterative operation on the iterative scatter distribution I_{sc}(i), thereby further improving calculation efficiency through upsampling and downsampling.

S8: Perform, based on the iterative scatter distribution I_{sc}(i) of the object, scatter correction on the irradiation intensity distribution I of the X-ray after passing through the object.

Specifically, after the iterative scatter distribution I_{sc}(i) of the object after the iteration is obtained based on step S7, an X-ray distribution Iₚᵣ without object scattering may be obtained by subtracting the iterative scatter distribution I_{sc}(i) of the object from the irradiation intensity distribution I of the X-ray after passing through the object, that is, Iₚᵣ=I-I_{sc}(i).

Except for the foregoing steps in this embodiment, the remaining steps are the same as those in the first embodiment. Details are not described herein again.

### Third embodiment

Based on the foregoing scatter correction method, the third embodiment of the present invention further provides a scatter correction system. As shown in FIG. 5, the scatter correction system includes one or more processors 21 and a memory 22. The memory 22 is coupled to the processor 21 and is configured to store one or more programs. When the one or more programs are executed by the one or more processors 21, the one or more processors 21 are enabled to implement the scatter correction method in the foregoing embodiment.

The processor 21 is configured to control an overall operation of the scatter correction system, to complete all or part of the steps of the foregoing scatter correction method. The processor 21 may be a central processing unit (CPU), a graphics processing unit (GPU), a field-programmable gate array (FPGA), an application-specific integrated circuit (ASIC), a digital signal processing (DSP) chip, or the like. The memory 22 is configured to store various types of data to support operations in the scatter correction system. The data may include, for example, instructions of any application program or method for operation in the scatter correction system, and data related to the application program. The memory 22 may be implemented by any type of volatile or non-volatile storage device or a combination thereof, for example, a static random access memory (SRAM), an electrically erasable programmable read-only memory (EEPROM), an erasable programmable read-only memory (EPROM), a programmable read-only memory (PROM), a read-only memory (ROM), a magnetic memory, and a flash memory.

In an exemplary embodiment, the system may be specifically implemented by a computer chip or an entity, or implemented by a product having a certain function, and is configured to perform the foregoing scatter correction method, and achieve the same technical effect as the foregoing method. A typical embodiment is a computer. Specifically, the computer may be, for example, a personal computer, a laptop computer, an on-board human-computer interaction device, a cellular phone, a camera phone, a smartphone, a personal digital assistant, a media player, a navigation device, an email device, a game console, a tablet computer, a wearable device, or any combination of these devices.

In another exemplary embodiment, the present invention further provides a computer-readable storage medium storing program instructions. The program instructions, when executed by a processor, implement the steps of the scatter correction method in any one of the foregoing embodiments. For example, the computer-readable storage medium may be the foregoing memory storing the program instructions, and the foregoing program instructions may be executed by the processor of the system to complete the foregoing scatter correction method, and achieve the same technical effect as the foregoing method.

Based on the above, the scatter correction method and system provided in embodiments of the present invention have the following beneficial effects:
1. The shape factors of scatter kernels for different thickness groups applicable to a full-ring detector are obtained through simulation. This extends a scatter deconvolution method from a conventional wide-fan-beam CT or CBCT scenario to a static CT scenario with a full-ring circular detector structure.
2. The irradiation intensity distributions of the X-ray before and after passing through the object can be used as a basis for grouping the thicknesses of the object, and then an overall scatter distribution of the object is obtained through scatter weighting of different thickness groups.
3. A scanning imaging mode of single-source alternate exposure for static CT and multi-source simultaneous exposure is supported.
4. Downsampling, upsampling, and the CUDA acceleration algorithm are used to accelerate scatter calculation to improve calculation efficiency.

The scatter correction method and system provided in the present invention are described in detail above. For a person of ordinary skill in the art, any apparent change made to the present invention without departing from the essential content of the present invention constitutes infringement of the patent right of the present invention and bears a corresponding legal responsibility.

## Claims

1. A scatter correction method, comprising the following steps:
obtaining an irradiation intensity distribution I₀ of an X-ray before passing through an object and an irradiation intensity distribution I of the X-ray after passing through the object;
calculating thicknesses t=I/I₀ of the object at different locations, and dividing the thicknesses of the object into n groups based on a preset thickness grouping boundary value, wherein n is a positive integer;
obtaining, based on thickness groups of the object, the irradiation intensity distribution I, and an X-ray attenuation index, irradiation intensity distributions S corresponding to different thickness groups, wherein S∈(S1, S2, ..., and Sn);
calculating, based on the thickness groups of the object and the irradiation intensity distributions S corresponding to different thickness groups and based on amplitude factors corresponding to different thickness groups, products A of the amplitude factors corresponding to different thickness groups and the irradiation intensity distribution I of the X-ray after passing through the object, wherein A∈(A1, A2, ..., and An);
obtaining shape factors F corresponding to different thickness groups of the object through simulation in advance, wherein F∈(F₁, F₂, ..., and Fₙ);
performing a two-dimensional convolution operation on A and the shape factors F corresponding to different thickness groups of the object, to obtain a scatter distribution I_{SC} of the object through superposition; and
performing, based on the scatter distribution I_{SC} of the object, scatter correction on the irradiation intensity distribution I of the X-ray after passing through the object.

2. The scatter correction method according to claim 1, wherein the performing, based on the scatter distribution I_{SC} of the object, scatter correction on the irradiation intensity distribution I of the X-ray after passing through the object is replaced by:
performing an iterative operation on the scatter distribution I_{SC} of the object to obtain an iterative scatter distribution I_{sc}(i) of the object, wherein i refers to a quantity of iterations; and
performing, based on the iterative scatter distribution I_{sc}(i) of the object, scatter correction on the irradiation intensity distribution I of the X-ray after passing through the object.

3. The scatter correction method according to claim 2, wherein the performing an iterative operation on the scatter distribution I_{SC} of the object to obtain an iterative scatter distribution I_{sc}(i) of the object specifically comprises:
obtaining a ray distribution Iₚᵣ(i-1) and the irradiation intensity distribution I₀ before the X-ray passes through the object, wherein Iₚᵣ(i-1)=I-I_{sc}(i-1), I_{sc}(0)=0, and Iₚᵣ(0)=I;
using Iₚᵣ(0) and I₀ as an input to obtain an iterative scatter distribution I_{sc}(1) of the object through calculation;
obtaining a new ray distribution Iₚᵣ(i) through calculation, wherein Iₚᵣ(i)=Iₚᵣ(i-1)+λ[I_{sc}(i-1)-I_{sc}(i)], λ is a relaxation factor that controls and adjusts a speed of iterative convergence, and a value of λ ranges from 0.6 to 0.8;
determining whether a current quantity of iterations reaches a preset value;
still performing iteration if the quantity of iterations does not reach the preset value, until the quantity of iterations reaches the preset value, and determining whether convergence is achieved if the quantity of iterations reaches the preset value; and
outputting the currently calculated iterative scatter distribution I_{sc}(i) if the convergence is achieved, and still performing, if the convergence is not achieved, a next iteration to obtain a scatter distribution I_{sc}(i+1), and simultaneously updating Iₚᵣ(i+1) until the convergence is achieved.

4. The scatter correction method according to claim 3, wherein the determining whether convergence is achieved specifically comprises:
obtaining a ratio of a difference between a current iterative scatter distribution I_{sc}(i) and a previous iterative scatter distribution I_{sc}(i-1) to I_{sc}(1);
comparing the ratio with a preset coefficient ε; and
determining that the convergence is achieved if the ratio is less than the preset coefficient ε, and determining that the convergence is not achieved if the ratio is not less than the preset coefficient ε.

5. The scatter correction method according to claim 3, wherein
a convergence degree of the iterative scatter distribution I_{sc}(i) is positively correlated with the quantity of iterations of the iterative scatter distribution I_{sc}(i).

6. The scatter correction method according to claim 2, wherein
the irradiation intensity distributions I₀ and I are downsampled, and the scatter distribution I_{SC} of the object is upsampled after the two-dimensional convolution operation.

7. The scatter correction method according to claim 6, wherein
the iterative operation is performed on the iterative scatter distribution I_{sc}(i) by using a CUDA acceleration algorithm.

8. The scatter correction method according to any one of claims 1 to 7, wherein
an exposure mode of the X-ray is alternate exposure of a single X-ray source in static CT.

9. The scatter correction method according to any one of claims 1 to 7, wherein
an exposure mode of the X-ray is simultaneous exposure of a plurality of X-ray sources in static CT.

10. A scatter correction system, comprising a processor and a memory, wherein the processor reads a computer program in the memory to perform the following operations:
obtaining an irradiation intensity distribution I₀ of an X-ray before passing through an object and an irradiation intensity distribution I of the X-ray after passing through the object;
calculating thicknesses t=I/I₀ of the object at different locations, and dividing the thicknesses of the object into n groups based on a preset thickness grouping boundary value, wherein n is a positive integer;
obtaining, based on thickness groups of the object, the irradiation intensity distribution I, and an X-ray attenuation index, irradiation intensity distributions S corresponding to different thickness groups, wherein SE(S1, S2, ..., and Sn);
calculating, based on the thickness groups of the object and the irradiation intensity distributions S corresponding to different thickness groups and based on amplitude factors corresponding to different thickness groups, products A of the amplitude factors corresponding to different thickness groups and the irradiation intensity distribution I of the X-ray after passing through the object, wherein AE(A1, A2, ..., and An);
obtaining shape factors F corresponding to different thickness groups of the object through simulation in advance, wherein F∈(F₁, F₂, ..., and Fₙ);
performing a two-dimensional convolution operation on A and the shape factors F corresponding to different thickness groups of the object, to obtain a scatter distribution I_{SC} of the object through superposition; and
performing, based on the scatter distribution I_{SC} of the object, scatter correction on the irradiation intensity distribution I of the X-ray after passing through the object.
